# EUROPÄISCHE PATENTSCHRIFT

(11) **EP 4 511 093 B1**
(45) Veröffentlichungstag und Bekanntmachung des Hinweises auf die Patenterteilung: **15.04.2026**
(21) Anmeldenummer: 23721605.6
(22) Anmeldetag: 19.04.2023
(51) Int. Cl.: B01D 46/44, A61M 13/00

(54) **INSUFFLATOR MIT VORRICHTUNG ZUR ERFASSUNG DER FILTERBELEGUNG**
INSUFFLATOR COMPRISING A DEVICE FOR DETECTING FILTER CLOGGING
INSUFFLATEUR COMPRENANT UN DISPOSITIF DE DÉTECTION DE COLMATAGE DE FILTRE

(30) Priorität: 20.04.2022 DE 102022001357
(43) Veröffentlichungstag der Anmeldung: 26.02.2025
(73) Patentinhaber: W.O.M. World of Medicine GmbH, 10587 Berlin (DE)
(72) Erfinder: KLUGE, Tobias, 12555 Berlin (DE); LANGEN, Fabian, 10407 Berlin (DE); CARSTENS, Jan Hendrik, 10409 Berlin (DE)
(74) Vertreter: Gulde & Partner
(86) Internationale Anmeldenummer: PCT/EP2023/060164
(87) Internationale Veröffentlichungsnummer: WO 2023/203090

(56) Entgegenhaltungen:
- EP-A1- 3 506 303
- WO-A1-2021/121462
- WO-A2-2007/050516
- US-A- 5 246 419
- US-A1- 2015 112 246

## Beschreibung

### Erfindungsgegenstand

Die vorliegende Erfindung betrifft einen Insufflator mit einer verbesserten Erfassung der Filterbelegung und Auswertung der Daten, um eine längere Standzeit des Filters oder eine gleichbleibende Funktion zu ermöglichen.

### Stand der Technik

Insufflatoren sind in mannigfaltiger Ausführung aus dem Stand der Technik bekannt. Insufflatoren weisen üblicherweise einen Schlauch auf, durch den ein medizinisches Gas in eine Körperhöhle (z. B. ein Abdomen) eingeleitet wird. Das Gas erzeugt einen Überdruck, welcher die Körperhöhle aufdehnt, damit ausreichend Platz für die visuelle Inspektion bzw. den therapeutischen Eingriff bzw. die medizinische Prozedur besteht. Einige Ausführungsformen ermöglichen das Absaugen des Gases aus der Körperhöhle über einen zweiten Schlauch. Diese Ausführungsformen können auch einen Durchfluss des Gases durch die Körperhöhle ermöglichen, z.B. um erwärmtes Gas zuzuführen und ein Auskühlen des Patienten zu verhindern. Häufiger werden derartige Ausführungsformen bei therapeutischen Eingriffen mittels Elektrochirurgie oder Laser genutzt, um die Sicht beim Eingriff zu gewährleisten und gesundheitsschädliche Rauchgase abzuführen. Im Sinne einer Ressourcenschonung werden diese Gase gefiltert und wieder zugeleitet um den Druckabfall durch das Absaugen zu kompensieren und die Körperhöhle aufgedehnt zu lassen.

Weiterhin ist im Zuge des Gesundheitsschutzes der Anwender eine Filterung des aus der Körperhöhle abgesaugten Gases, welches in die Umgebung abgegeben wird, erforderlich.

In der Praxis hat es sich als schwierig herausgestellt, die Filter für eine im Vorhinein nicht festgelegte Dauer der Operation auszulegen. Die Möglichkeit, den Filter sehr groß zu dimensionieren, damit er lange seine Filterwirkung erfüllen kann, ist kostenintensiv und behindert durch einen großen und schweren Filter. Eine zu kleine Dimensionierung ist problematisch, weil mit fortschreitender Nutzung die Filterwirkung nachlässt, bzw. der Durchlasswiderstand sich erhöht. Dies drückt sich dadurch aus, dass sich die Sicht durch fehlende Absaugleistung verschlechtert.

Es gibt vielfältige Lösungen für die Erfassung der Belegung eines Filters, die auf dem unterschiedlichen Druck vor und hinter dem Filter basieren. Hierbei werden die, durch den mit der Filterbeladung steigenden Durchlasswiderstand hervorgerufenen Druckunterschiede ausgewertet.

WO2007/050516A2 beschreibt ein System zur Steuerung der Zufuhr eines Aufweitungsmediums (z. B. eines Insufflationsgases) von einer Aufweitungsmedienquelle zu einer endoskopischen Vorrichtung, um eine übermäßige Entlüftung und/oder Verschwendung von Aufweitungsmedien zu verhindern.

EP3506303A1 offenbart chirurgische Systeme und deren Absaugvorrichtungen. Die beschriebenen chirurgischen Rauchabsauger sind so konfiguriert, dass sie sowohl Rauch als auch Flüssigkeit und/oder Partikel von einer Operationsstelle abtransportieren.

WO2021/121462A2 beschreibt ein für chirurgische Eingriffe bestimmtes Schlauchset mit Gerätekupplung, das eine Anordnung von Wasserabscheider und Filter in einem einteiligen Gehäuse enthält.

US20210207833A1 "Device and method for monitoring HVAC air filter" ist eine sehr aktuelle Anmeldung die mittels der beschriebenen Differentialdruckmessung eine Vorhersage der Filterbeladung beschreibt. Die beschriebenen HVAC-Filter sind technisch den im Insufflator eingesetzten Filtern sehr ähnlich.

In der Anmeldung WO2019186501A1 "Method of sensing particulate from smoke evacuated from a patient, adjusting the pump speed based on the sensed information, and communicating the functional parameters of the system to the hub" wird die Auswertung von Rauchpartikeln zur Steuerung einer Pumpleistung beschrieben. In den Absätzen [0264] f. bzw. als "Example 41" ist grob eine Funktionalität mit Drucksensoren beschrieben wie die erfindungsgemäße Lösung sie vorsieht, jedoch als Idee ohne jede technische Lehre, wann welche Aktion zum gewünschten Ergebnis führt. Die Steuerung erfolgt für das raucherzeugende Medizingerätes (RF generator power request) und Absatz [0282] beschriebt die Möglichkeit einen Alarm zu erzeugen. Es werden jedoch keine Auswertungen von Druckmessungen gemacht, sondern von Sensoren, die Partikelgröße und -konzentration ermitteln.

Andere Schriften zum Stand der Technik sind unter anderen US2927659A "Dust collector", wo bereits die Differenzdruckmessung zur Erfassung der Filterbeladung beschrieben ist. Weiterhin sind Verfahren zur Überwachung der Filterbelegung beschrieben in NL2024196B1 "A monitoring system, method and vehicle comprising such a system, for detecting clogging through fouling of an air filter of an internal combustion engine."; EP2960484A1 "System for detecting an air filter condition, in particular for combustion engines" sowie US11202982B2 "Air filter condition sensing".

Somit ist sehr nah am Stand der Technik eine Lösung zur Erhöhung des Durchflusses durch die aufgedehnte Körperhöhle mittels einem über Differentialdruck erkannten Durchflusswiderstand, wobei dieser durch steigende Filterbeladung zunimmt, um eine gleichmäßige, patientenschonende Aufdehnung zu gewährleisten. Diese Lösung kann bereits durch einen Flowsensor (Durchflussmengen-Sensor) als Sensor und eine Regelung zur Stabilisierung der Flowmenge gewährleistet werden, wobei bei der beschriebenen, mit der Methode des Differentialdruckes gesteuerten Flowerhöhung sicherstellt ist, dass kein erhöhter Druck (es geht hier ja teilweise um sehr niedrige Drücke um 15 mmHg) in der Körperhöhle erzeugt wird, damit die erhöhte Saugleistung durch den erhöhten Durchflusswiderstand gerade kompensiert und nicht überkompensiert wird und auch Leckagen keinen negativen Einfluss auf die Größe der Aufdehnung haben.

Weiterhin ist sehr nah am Stand der Technik, dass durch die Auswertung der Differenzdruckwerte über die Zeit, indem sprunghafte Änderungen oder eine Auswertung der ersten Ableitung der Druckwerte z.B. anhand der Festlegung eines Schwellwertes eine Unterscheidung zwischen Verlegung/Okklusion und dem Zusetzen des Filters erfolgen kann.

Ebenfalls offenbart ist eine Nutzung des Differenzdruckes bzw. eines Schwellwertes für den Druck als Warnsignal für einen Filteraustausch. Dem folgend solle eine Abschätzung der Rest-Standzeit möglich sein.

### Erfindungsgemäße Lösung

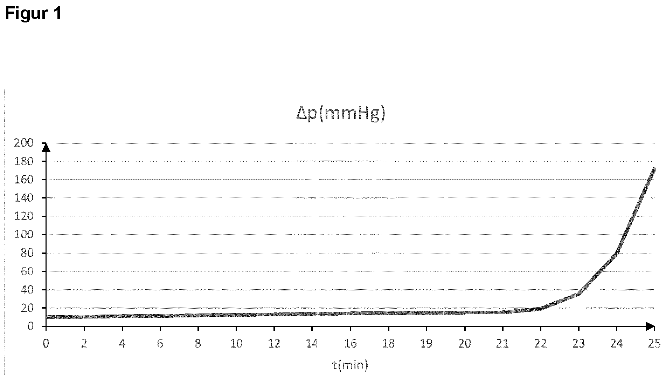

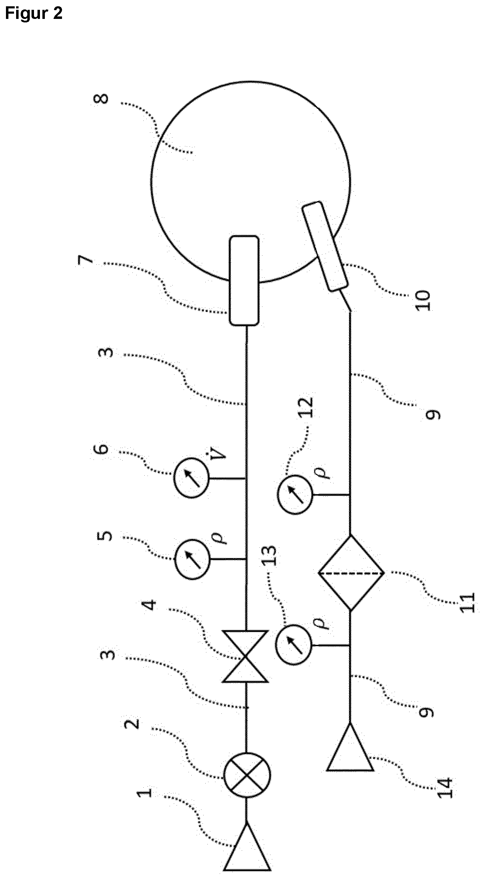

Die Erfindung ist in den angehängten Ansprüchen definiert. Im Rahmen der Entwicklung von Insufflatoren hat sich herausgestellt, dass die für den Betrieb von Insufflator eingesetzten Filtersysteme die detektierten Druckverläufe beim Zusetzen des Filters ein sehr spezifisches Verhalten zeigen, nämlich zunächst einen langsamen linearen Anstieg des Differenzdruckes mit einem dann exponentiell zunehmendem Differenzdruck bis hin zum Verschluss des Filters. Die beim Insufflator eingesetzten Filtersysteme bestehen aus einer wasserabweisenden Schicht, gefolgt von einer Partikel-filtrierenden und einer chemisch bindenden Schicht, wobei die Reihenfolge der letzten beiden genannten Schichten auch vertauscht sein kann. Ebenso ist es möglich, dass die Partikel-filtrierende und die chemisch bindende Funktion in einer Schicht realisiert sind. Dieser Druckverlauf über die Zeit ist modellhaft in Figur 1 dargestellt. Dargestellt ist der gemessene Druckverlust, also die Druckdifferenz des Gasstroms vor und nach dem Filter bei einem Gasstrom von 25 L/min). Der Druckverlust mit einem frischen Filter beträgt ca. 10 mmHg. Mit der Zeit steigt der Druckverlust annähernd linear an. Nach ca. 20 Minuten beträgt der Druckverlust ca. 15 mmHg. Danach wächst der Druckverlust exponentiell an und erreicht nach ca. 23,5 Minuten ca. 50 mmHg. Ein Druckverlust von 50 mmHg ist für eine Insufflation nicht mehr akzeptabel. Im Normalfall wird man bereits Druckdifferenzen von mehr als 35 mmHg vermeiden wollen. Entscheidend für einige Ausführungsformen der Erfindung ist die Erkennung des Beginns des exponentiellen Anstiegs des Druckverlustes. Wichtig zu wissen ist, dass die Filter auch ohne rauchgaserzeugende Operationsmodi langsam zur Okklusion neigen, vermutlich aufgrund der unvermeidlichen Feuchtigkeit aus den aufgedehnten Körperhöhlen.

Da bei einer zu hohen Okklusion und somit zu hohem Druckverlust eine Unterbrechung der Prozedur und ein Filterwechsel unumgänglich ist, besteht ein Bedarf an einer realistischen Vorhersage der Restnutzungsdauer.

Nach den im Stand der Technik beschriebenen Methoden liefert die Vorhersage einer Standzeit bzw. Restnutzungsdauer eine falsche, nämlich überraschend verkürzte Standzeit, was im OP-Verlauf eine kritische Situation hervorrufen kann.

Die Offenbarung betrifft daher einen Insufflator für die minimal-invasive Chirurgie, enthaltend
a) Gasanschluss mit Proportionalventil,
b) einen ersten Trokar
c) eine Gaszuführleitung für Gas aus dem Gasanschluss mit Druckmessung, Filter und Anschluss an den ersten Trokar,
d) eine Absaugvorrichtung mit regelbarer Absaugleistung,
e) einen zweiten Trokar mit Absaugschlauch, angeschlossen an die Absaugvorrichtung mit regelbarer Absaugleistung,
f) einen Filter zur Reinigung des abgesaugten Gases, der zwischen dem zweiten Trokar und der Absaugvorrichtung positioniert ist,
h) mindestens einen Drucksensor der in Richtung des Gasstromes vor oder hinter dem Filter positioniert ist,
j) eine elektronische Regelungseinheit,
k) eine Anzeigeeinrichtung,
wobei der Insufflator mindestens einen Gasdruck¬sensor enthält, der die Gasdruckdifferenz vor und hinter dem Filter durch Auslenkung einer Membran messen kann.

Die elektronische Regelungseinheit bestimmt auf Basis der gemessenen Werte der Drucksensoren und/oder der Gasflusssensoren den Beladungszustand des Filters, berechnet daraus die erwartete Restlaufzeit und zeigt die verbleibende Restlaufzeit auf einer geeigneten Anzeigeeinrichtung an.

Ferner wird durch die elektronische Regelungseinheit ein Alarm ausgelöst, wenn das lineare Wachstum der Druckdifferenz in ein exponentielles Wachstum übergeht oder wenn der linear sinkende Gasfluss in einen exponentiell sinkenden Gasfluss übergeht.

Darüber hinaus wird durch die elektronische Regelungseinheit ein Signal auslöst, welches den Anwender temporär zur Anwendung einer Betriebsweise ohne Rauchgaserzeugung auffordert, wenn das lineare Wachstum der Druckdifferenz in ein exponentielles Wachstum übergeht oder wenn der linear sinkende Gasfluss in einen exponentiell sinkenden Gasfluss übergeht.

Üblicherweise wird der Druck im Absaugschlauch vor und hinter dem Filter gemessen. Die Angabe der Positionen "vor dem Filter" und "nach dem Filter" bezieht sich auf die Strömungsrichtung im Absaugschlauch. Die Messung kann beispielsweise über mindestens zwei Gasdrucksensoren erfolgen, von denen mindesten ein Gasdrucksensor vor dem Filter und mindestens ein Gasdrucksensor nach dem Filter angeordnet ist. Die Druckdifferenz kann aus den Messwerten berechnet und der Verlauf der Druckdifferenz über die Zeit überwacht werden.

Im einfachsten Fall kann der Verlauf der Filterokklusion auch über einen einzigen Gasdrucksensor gemessen werden. Mit Zunahme der Okklusion wird bei konstantem Eingangsdruck und konstantem Gasvolumenstrom der Druck vor dem Filter größer, während der Druck nach dem Filter abnimmt. Bei variablen Druck- und Volumenstrombedingungen ist das Druckverhalten zwar komplexer, kann aber aufgrund der klaren Korrelation zwischen Druck und Okklusionsgrad modelliert werden. Damit erlaubt auch ein einziger Gasdrucksensor vor oder nach dem Filter eine Messung des Okklusionsverhaltens und eine Berechnung der Reststandzeit.

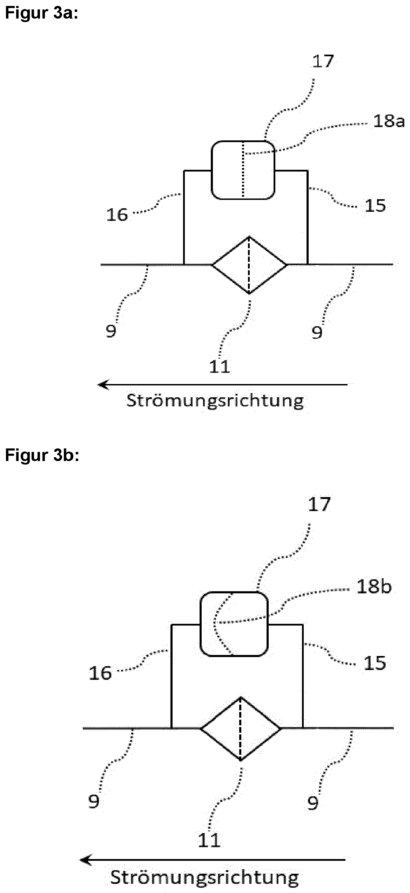

Erfindungsgemäße wird für die Erfassung der Druckdifferenz das Messprinzip angewandt, wobei pneumatisch eine Differenz gebildet wird, indem eine elastische Membran ausgelenkt wird und dann die Auslenkung mit einem Weg- oder Winkelgeber abgenommen wird. Dies bedeutet, dass beide Drücke auf unterschiedlichen Seiten des Filters vom Filtergehäuse abgeleitet und über Kanäle zum Sensor mit der Membran geführt werden und dort mechanisch im Gerät die Auslenkung durch den Druckunterschied bestimmt wird (Figur 3 a/b). Die Erfassung der Auslenkung dieser Membran kann gemäß dem Stand der Technik beispielsweise durch mechanische Abtastung erfolgen. Alternativ kann die Auslenkung der Membran auch über den Stand der Technik hinausgehend auch optisch, z.B. über eine Ablenkung eines Lichtstrahles mittels eines auf der Membran befindlichen Spiegels auf eine CCD-Zeile, also nebeneinander angeordnete lichtempfindliche Elemente, erfolgen.

Erfindungsgemäß wird die berechnete Restnutzungsdauer des Filters durch eine Anzeigevorrichtung angezeigt. Die Anzeigevorrichtung kann im Display des Insufflators integriert oder durch ein externes Anzeigegerät realisiert sein. In jedem Fall wird für die Durchführenden der medizinischen Prozedur ein optischer und/oder akustischer Alarm gegeben. Der relevante Grenzwert für die Auslösung des Alarms kann am Gerät einstellbar sein. So kann beispielsweise ein erster Alarm ausgelöst werden, wenn das lineare Wachstum der Druckdifferenz in ein exponentielles Wachstum übergeht. Weiterhin kann ein zweiter Alarm ausgelöst werden, wenn die Druckdifferenz in den kritischen Bereich kommt (z.B. 35 - 50 mmHg).

Die Restnutzungsdauer kann über eine Auswertung der ermittelten Werte mit mathematischen Methoden stabilisiert werden, indem beispielsweise ein Verfahren zur Zeitreihenanalyse angewendet wird oder das oben beschriebene Verhalten bei den Druckwerten (linearer Anstieg, dann Übergang in exponentiell) wird über mathematische Methoden an die Messwerte angenähert (Parameterschätzung / *curve fit*)*.* Erfindungsgemäß wird also mit der Differenzdruckmessung und der Auswertung über die Zeit eine präzisere Voraussage der Reststandzeit bis zum Verstopfen des Filters sowie andere, die Standzeit verlängernde Änderungen in den Betriebsparametern möglich, wie sie weiter unten beschrieben werden.

In einer weiteren Ausführungsform der Erfindung wird der Beladungsgrad des Filters über den Gasfluss ermittelt. Bei konstanter Leistung der Absaugpumpe verändert sich der Gasfluss über die Zeit: Mit zunehmendem Beladungsgrad wird der Gasfluss naturgemäß geringer. Bei dem Modellaufbau gemäß Figur 1 liegt der Gasfluss zu Beginn der Messungen (bei frischem Filter) bei ca. 23 L/min. Nach 20 Minuten ist der Gasfluss auf ca. 20 L/min. gesunken. Entsprechend der Erhöhung der Druckdifferenz kommt es zu einer Absenkung des Gasflusses. Die Gasflussabsenkung ist ebenfalls anfänglich linear, später dann exponentiell. Im genannten Modellaufbau ist nach ca. 23 Minuten der Gasfluss annähernd linear auf unter 10 L/min gesunken und sinkt dann exponentiell weiter.

Analog zur Auswertung des Differenzdruckes kann daher die Ermittlung der Restnutzungsdauer durch Messung Auswertung des Gasflusses erfolgen. Entscheidend für einige Ausführungsformen der Erfindung ist die Erkennung des Beginns des exponentiellen Absinkens des Gasflusses. Ganz analog zu den oben geschilderten Ausführungsformen der Erfindung unter Ermittlung des Druckverlustes kann die Alarmauslösung anhand eines oder mehrerer Schwellwerte erfolgen, die naturgemäß abhängig sind von der Art des benutzten Filters und der zu erwartenden Rauchgaserzeugung. Diese werden vorab durch Messungen ermittelt. Alternativ kann eine sinnvolle Restnutzungsdauer bzw. Schwellwerte zur Auslösung eines Alarmes vom Hersteller vorgegeben oder vom Nutzer eingestellt werden und die oben beschriebene Methode der mathematischen Methoden Zeitreihenanalyse oder Parameterschätzung herangezogen werden, um abzuprüfen ob diese durch die ermittelte Restdauer überschritten wird und die Rauchgasabsaugung nur zu ermöglichen, wenn diese Bedingung erfüllt ist.

In einem weitergehenden Offenbarungsschritt erfolgt eine getrennte Darstellung der Restnutzungsdauer für normale Betriebsmodi (z.B. reine Insufflation zur visuellen Inspektion des Abdomens) und Rauchgasabsaugung (z.B. während operativen Eingriffen mit Elektrokoagulation). Diese werden aufgrund der Beladung zu einem bestimmten Zeitpunkt im Rausgasabsaugungsmodus und der im nicht-Rauchgasbetriebsarten getrennt ermittelt. Hierzu muss das das Gerät den jeweiligen Betriebsmodus erkennen und passt zwei getrennte Kurvenverläufe für die Restnutzungsdauer gemäß den oben bereits beschriebenen Methoden an und aktualisiert diese für die jeweils aktiven Modi. So können beispielsweise für einen bestimmen Insufflator mit einem bestimmten Filter die Kurvenverläufe der Druckdifferenz (analog zu Figur 1) für verschiedene Betriebsmodi experimentell bestimmt werden. Während einer Prozedur können dann, ausgehend von der aktuellen Druckdifferenz als Maß der Filterbeladung, die Restlaufzeiten für verschiedene Betriebsmodi errechnet werden (z.B. 5 min bei Elektrokoagulation bzw. 15 min bei visueller Inspektion).

Weiterführend können diese Werte oder auch nur einer der Werte auch genutzt werden, um einen Alarm für einen Wechsel des Filters oder des Schlauchsets zu erzeugen. Offenbarungsgemäß wäre dies beispielsweise, wenn die ermittelte Restnutzungsdauer der Betriebsmodi nicht-Rauchgasabsaugung kleiner ist als 1/4 der Auslegungsstandzeit - und/oder die ermittelte Restnutzungsdauer des Rauchgasabsaugungsmodus kleiner ist als 1/10 der Auslegungsstandzeit - andere Festlegungen sind abhängig von den Nutzungsszenarien möglich. Dieser Alarm würde gegeben für eine medizinischen Prozedur von 15 Minuten (Auslegungsstand zeit) bei einer Restnutzungsdauer von unter 3,75 Minuten für Betriebsmodi nicht-Rauchgasabsaugung und/oder unter 1,5 Minuten für den Rauchgasabsaugungsmodus.

Offenbarungsgemäß wird die Druckmessung pneumatisch gekoppelt an den Filter. Das bedeutet eine Platzierung des Sensors im Gerät und ein fluidische Verbindung zum Messort. Dies hat den Vorteil, dass die sterilen Einmalartikel (Schlauchset mit Filter) ohne elektronische Komponenten auskommen und Kosten, Sterilisierung und Umweltfreundlichkeit positiv beeinflusst werden.

Dieser pneumatische Sensorkanal muss bestimmte Eigenschaften aufweisen. Die Druckmessung vor dem Filter erfolgt mit mindestens einem zusätzlichen Sensor, ansonsten werden vorhandene Druckwerte genutzt (Absaugdruck, Inflowdruck / Treiberdruck), die der Insufflator für die Regelung des Druckes in der Körperhöhle benötigt. Der Sensorkanal, der vor dem zu beobachtenden Filter abzweigt, hat ebenfalls einen Filter, um eine teilweise Verlegung oder ein Zusetzen zu verhindern, da dies eine Verfälschung der erfassten Druckwerte bewirken würde. Ebenso muss der Durchmesser im Bereich zwischen 0,1 und 5 mm liegen, damit ein zügiger Ausgleich des Druckes erfolgen kann.

Eine offenbarungsgemäße aus der Druckmessung abgeleitete Methode zur Standzeiterhöhung des Filters kann durch eine Pause der Absaugung bei der Rauchgaserzeugung erfolgen. Diese Pause kann einen Druckverlust in der Kavität bewirken, die nach ein paar Minuten wirksam wird, allerdings schnell wieder ausgeglichen ist in der Größenordnung von einigen Sekunden. Pausen bringen eher nicht viel zusätzliche Standzeit.

Offenbarungsgemäß ist ein Betriebsmodus, in dem Pausen beim Durchfluss gemacht werden, um den im Filter gesammelten Partikeln zu erlauben sich zu setzen und die Verlegung der Poren durch den Andruck des Fluidstromes zu unterbrechen. Dabei ist ein Puls-Pause-Verhältnis von 1 Minute Pause zu 10 Sekunden Durchfluss vorgesehen. Hierdurch erhöht sich die Nutzungsdauer des Filters, da in diesem Modus eine Weiterführung der medizinischen Prozedur möglich ist. Ebenfalls offenbarungsgemäß ist eine automatische Umschaltung in einen solchen Betriebsmodus, die durch einen Schwellwert im Differenzdruck ausgelöst werden kann. Weiterführend wird der bei Aktivierung des Betriebsmodus vorhandene Durchfluss (in sLm = Standard-Liter pro Minute) ausgewertet, um die Puls-Pause-Verhältnisse festzulegen - bei hohen Leckagen beispielsweise kürzere Pausen, um ein Zusammensacken der Aufdehnung zu minimieren. Hohe Leckagen ergeben sich aus der Lage des Arbeitspunktes der Regelung, also den Wertepaaren von geschätztem Kavitätendruck und in die Kavität eingeleitetem Durchfluss.

Offenbarungsgemäß und wirksamer ist ein Betriebsmodus, worin der Anwender für eine gewisse Dauer zu einer Vermeidung/Pause der Erzeugung von Rauchgas (also Lasertherapie/HF-Anwendung/etc.) aufgefordert wird und nach dieser Zeit wieder ein optisches oder akustisches Signal bekommt, dass er wie bisher Rauchgas erzeugen kann. Der Insufflator kann in dieser Zeit ein Trockenlaufen des Filters mit einer veränderten, höheren Durchflussmenge bewirken, da ein feuchter Filter den Durchflusswiderstand besonders erhöht. Hierdurch kann eine Erhöhung der bzw. eine Verlängerung der Zeit einer Filterwirkung für die medizinische Prozedur bzw. der Filter-Standzeit erreicht werden. Eine offenbarungsgemäße Dauer wären einige Minuten (mindestens 1,5 Minuten, optimal 5 Minuten). Eine offenbarungsgemäße Durchflusserhöhung wäre eine Verdopplung der Durchflussmenge, genauer gefasst eine Durchflussmenge zwischen 5 sLm und 50 sLm. Mit einem Durchflussvolumen von 5 sLm wäre eine Kavität von ca. 5 L Volumen, wie es der aufgedehnte Abdomen ist, einmal in einer Minute ausgetauscht mit unbelastetem Aufdehnungsmedium und nach dem initialen Austausch setzt in den nächsten Minuten der Filtertrocknungseffekt ein.

Eine weitere offenbarungsgemäße Option bei Nutzung eines befeuchteten und geheizten Schlauchsets ist es, für diesen Filtertrocknungs-Betriebsmodus die Heizung herunterzuregulieren oder auszuschalten, damit die Wärme an dem Befeuchtungsmedium nicht zusätzlich Feuchtigkeit in der Körperhöhle verdunstet und nach der Beendigung des Betriebsmodus die Heizung wieder einzuschalten.

### Bezugszeichenliste

(1) Gasanschluss
(2) Proportionalventil
(3) Gaszuführleitung
(4) Volumenstromregelung
(5) Drucksensor (Zuführleitung)
(6) Volumenstromsensor (Zuführleitung)
(7) erster Trokar
(8) Kavität
(9) Absaugschlauch
(10) zweiter Trokar
(11) Filter
(12) Gasdrucksensor (in Strömungsrichtung vor dem Filter)
(13) Gasdrucksensor (in Strömungsrichtung hinter dem Filter)
(14) Absaugeinrichtung (regelbar)
(15) Abzweigleitung vor dem Filter zur Druckmessmembran
(16) Abzweigleitung nach dem Filter zur Druckmessmembran
(17) Gehäuse der Druckmessmembran
(18a) Druckmessmembran (neutral, bei gleichem Druck vor und hinter dem Filter)
(18b) Druckmessmembran (ausgelenkt, bei höherem Druck vor dem Filter)

Die Offenbarung umfasst auch Verfahren zum Betrieb eines Insufflators für die minimal-invasive Chirurgie, enthaltend
a) Gasanschluss mit Proportionalventil,
b) einen ersten Trokar
c) eine Gaszuführleitung für Gas aus dem Gasanschluss mit Druckmessung, Filter und Anschluss an den ersten Trokar,
d) eine Absaugvorrichtung mit regelbarer Absaugleistung,
e) einen zweiten Trokar mit Absaugschlauch, angeschlossen an die Absaugvorrichtung mit regelbarer Absaugleistung,
f) einen Filter zur Reinigung des abgesaugten Gases, der zwischen dem zweiten Trokar und der Absaugvorrichtung positioniert ist,
h) mindestens einen Drucksensor der in Richtung des Gasstromes vor oder hinter dem Filter positioniert ist,
j) eine elektronische Regelungseinheit,
k) eine Anzeigeeinrichtung,

wobei der Insufflator mindestens einen Gasdruck¬sensor enthält, der die Gasdruckdifferenz vor und hinter dem Filter durch Auslenkung einer Membran messen kann,
insbesondere:
   Verfahren zum Betrieb eines Insufflators, wobei der Druck im Absaugschlauch vor und hinter dem Filter kontinuierlich gemessen wird, aus den gemessenen Werten die Druckdifferenz bestimmt wird und wobei aus der Veränderung der Druckdifferenz der Beladungszustand des Filters ermittelt und angezeigt wird.

Verfahren zum Betrieb eines Insufflators, wobei durch die elektronische Regelungseinheit ein Alarm ausgelöst wird, wenn das lineare Wachstum der Druckdifferenz in ein exponentielles Wachstum übergeht oder wenn der linear sinkende Gasfluss in einen exponentiell sinkenden Gasfluss übergeht.

Verfahren zum Betrieb eines Insufflators, wobei durch die elektronische Regelungseinheit ein Alarm ausgelöst wird, wenn das lineare Wachstum der Druckdifferenz in ein exponentielles Wachstum übergeht sowie optional ein zweiter Alarm ausgelöst wird, wenn die Druckdifferenz in den kritischen Bereich kommt (z.B. 35 - 50 mmHg).

Verfahren zum Betrieb eines Insufflators, wobei die Restnutzungsdauer des Filters über eine Auswertung der ermittelten Werte mit mathematischen Methoden erfolgt, indem beispielsweise ein Verfahren zur Zeitreihenanalyse angewendet wird oder das oben beschriebene Verhalten bei den Druckwerten (linearer Anstieg, dann Übergang in exponentiell) wird über mathematische Methoden an die Messwerte angenähert (Parameterschätzung / *curve fit*)*.*

Verfahren zum Betrieb eines Insufflators, wobei die elektronische Regelungseinheit den Anwender durch ein Signal zu Pausen beim Durchfluss auffordert, um den im Filter gesammelten Partikeln zu erlauben sich zu setzen und die Verlegung der Poren durch den Andruck des Fluidstromes zu unterbrechen.

Verfahren zum Betrieb eines Insufflators, wobei die elektronische Regelungseinheit eine automatische Umschaltung in einen Betriebsmodus auslöst, die Pausen beim Durchfluss erlaubt.

Verfahren zum Betrieb eines Insufflators, wobei der Anwender durch ein erstes Alarmsignal für eine gewisse Dauer zu einer Vermeidung/Pause der Erzeugung von Rauchgas (also Lasertherapie/HF-Anwendung/etc.) aufgefordert wird und nach dieser Zeit wieder ein optisches oder akustisches Signal bekommt, dass er wie bisher Rauchgas erzeugen kann.

## Patentansprüche

1. Insufflator für die minimal-invasive Chirurgie, enthaltend
a) Gasanschluss (1) mit Proportionalventil (2),
b) einen ersten Trokar (7)
c) eine Gaszuführleitung (3) für Gas aus dem Gasanschluss (1) mit Druckmessung, Filter und Anschluss an den ersten Trokar (7),
d) eine Absaugvorrichtung mit regelbarer Absaugleistung,
e) einen zweiten Trokar (10) mit Absaugschlauch (9), angeschlossen an die Absaugvorrichtung mit regelbarer Absaugleistung (14),
f) einen Filter (11) zur Reinigung des abgesaugten Gases, der zwischen dem zweiten Trokar (10) und der Absaugvorrichtung (14) positioniert ist, h) mindestens einen Drucksensor (12, 13) der in Richtung des Gasstromes vor (12) oder hinter (13) dem Filter (11) positioniert ist,
j) eine elektronische Regelungseinheit,
k) eine Anzeigeeinrichtung
wobei die elektronische Regelungseinheit die Absaugvorrichtung auf Basis der gemessenen Werte der Drucksensoren den Beladungszustand des Filters bestimmt und daraus die erwartete Restlaufzeit berechnet und wobei die Anzeigeeinrichtung die verbleibende Restlaufzeit anzeigt
und wobei durch die elektronische Regelungseinheit ein Alarm ausgelöst wird, wenn das lineare Wachstum der Druckdifferenz in ein exponentielles Wachstum übergeht,
**dadurch gekennzeichnet, dass**
der mindestens eine Drucksensor (12, 13) die Gasdruckdifferenz vor (12) und hinter (13) dem Filter durch Auslenkung einer Membran (18a, 18b) messen kann.

2. Insufflator gemäß Anspruch 1, wobei der Insufflator ein Signal auslöst, welches den Anwender temporär zur Anwendung einer Betriebsweise ohne Rauchgaserzeugung auffordert, wenn
das lineare Wachstum der Druckdifferenz in ein exponentielles Wachstum übergeht.

3. Insufflator gemäß Anspruch 1 oder 2, wobei die Auswertung der Messdaten auf Basis von Zeitreihenanalysen und/oder Parameterschätzung / curve fit erfolgt.

4. Insufflator gemäß Anspruch 1, wobei die Regelungseinheit die verbleibende Restlaufzeit für den Fall einer Betriebsweise ohne Rauchgaserzeugung und eine Restlaufzeit für den Fall einer Betriebsweise mit Rauchgaserzeugung anzeigt.

5. Insufflator gemäß mindestens einem der Ansprüche 1 bis 4, welcher mindestens einen Alarm auslöst, wenn die ermittelte Restlaufzeit des Filters (11) ein oder mehrere vorher festgelegte Zeitdauern unterschreitet.

6. Insufflator gemäß Anspruch 5, wobei der Zeitraum einer Betriebsweise ohne Rauchgaserzeugung mindestens 1,5 Minuten, bevorzugt ca. 5 Minuten bei einem Volumenstrom von 5 sLm und 50 sLm beträgt.

7. Insufflator gemäß mindestens einem der Ansprüche 1 bis 6, wobei der Filter (11) mehrere Schichten enthält, wovon mindestens eine Schicht eine wasserabweisende Schicht ist, wovon mindestens eine Schicht eine Partikel-filtrierenden Schicht ist und wovon mindestens eine Schicht eine chemisch bindende Schicht ist.

8. Insufflator gemäß Anspruch 7, wobei der Filter (11) drei Schichten enthält, wobei die in Strömungsrichtung erste Schicht eine wasserabweisende Schicht ist, gefolgt von einer Partikel-filtrierenden und einer chemisch bindenden Schicht in beliebiger Reihenfolge.

9. Insufflator gemäß Anspruch 8, wobei die Partikel-filtrierende und die chemisch bindende Funktion in einer einzigen Schicht realisiert sind.

## Claims

1. An insufflator for minimally invasive surgery, comprising
a) a gas connection (1) with a proportional valve (2),
b) a first trocar (7),
c) a gas supply line (3) for gas from the gas connection (1) with a pressure measurement, filter, and connection to the first trocar (7),
d) an extraction device with an adjustable extraction capacity,
e) a second trocar (10) with an extraction hose (9), connected to the extraction device with the adjustable extraction capacity (14),
f) a filter (11) for cleaning extracted gas, positioned between the second trocar (10) and the extraction device (14),
h) at least one pressure sensor (12, 13) positioned in the direction of a gas flow upstream (12) or downstream (13) of the filter (11),
j) an electronic control unit,
k) a display device,
wherein the electronic control unit determines a loading state of the filter based on measured values from the pressure sensors and calculates therefrom an expected remaining operating time, and wherein the display device displays a remaining operating time,
and wherein the electronic control unit triggers an alarm when the linear growth of a pressure difference transitions into exponential growth,
**characterized in that**
at least one pressure sensor (12, 13) is capable of measuring a gas pressure difference upstream (12) and downstream (13) of the filter by deflecting a diaphragm (18a, 18b).

2. The insufflator according to claim 1, wherein the insufflator triggers a signal that temporarily prompts a user to use an operating mode without flue gas generation when the linear growth of the pressure difference transitions into exponential growth.

3. The insufflator according to claim 1 or 2, wherein measurement data are evaluated based on time series analysis and/or parameter estimation/curve fitting.

4. The insufflator according to claim 1, wherein the control unit displays a remaining operating time in an operating mode without flue gas generation and a remaining operating time in an operating mode with flue gas generation.

5. The insufflator according to at least one of claims 1 to 4, which triggers at least one alarm when a determined remaining operating time of the filter (11) falls below one or more predetermined time periods.

6. The insufflator according to claim 5, wherein the operating time in the operating mode without flue gas generation is at least 1.5 minutes, preferably approximately 5 minutes, at a volume flow rate of 5 sLm and 50 sLm.

7. The insufflator according to at least one of claims 1 to 6, wherein the filter (11) comprises several layers, at least one of which is a water-repellent layer, at least one of which is a particle-filtering layer, and at least one of which is a chemically binding layer.

8. The insufflator according to claim 7, wherein the filter (11) comprises three layers, the first layer in the flow direction being a water-repellent layer, followed by a particle-filtering layer and a chemically binding layer in any order.

9. The insufflator according to claim 8, wherein particle-filtering and chemically binding functions are implemented in a single layer.

## Revendications

1. Insufflateur pour la chirurgie mini-invasive, comprenant
a) un raccord de gaz (1) avec une vanne proportionnelle (2),
b) un premier trocart (7),
c) une conduite d'alimentation en gaz (3) pour le gaz provenant du raccord de gaz (1) avec mesure de pression, filtre et raccordement au premier trocart (7),
d) un dispositif d'aspiration avec une puissance d'aspiration réglable,
e) un deuxième trocart (10) avec un tuyau d'aspiration (9), raccordé au dispositif d'aspiration avec une puissance d'aspiration réglable (14),
f) un filtre (11) destiné à l'épuration du gaz aspiré, positionné entre le deuxième trocart (10) et le dispositif d'aspiration (14),
h) au moins un capteur de pression (12, 13) positionné en amont (12) ou en aval (13) du filtre (11) dans le sens de l'écoulement du gaz,
j) une unité de régulation électronique,
k) un dispositif d'affichage
l'unité de régulation électronique déterminant l'état de charge du filtre sur la base des valeurs mesurées par les capteurs de pression et calculant à partir de celles-ci la durée de vie restante prévue, et le dispositif d'affichage affichant la durée de vie restante
et l'unité de régulation électronique déclenche une alarme lorsque la croissance linéaire de la différence de pression se transforme en une croissance exponentielle
**caractérisé en ce que**
le ou les capteurs de pression (12, 13) pouvant mesurer la différence de pression du gaz en amont (12) et en aval (13) du filtre par la déviation d'une membrane (18a, 18b).

2. Insufflateur selon la revendication 1, dans lequel l'insufflateur déclenche un signal qui invite temporairement l'utilisateur à utiliser un mode de fonctionnement sans production de gaz de combustion lorsque la croissance linéaire de la différence de pression passe à une croissance exponentielle.

3. Insufflateur selon la revendication 1 ou 2, pour lequel l'évaluation des données de mesure est effectuée sur la base d'analyses de séries temporelles et/ou d'estimation de paramètres / ajustement de courbe.

4. Insufflateur selon la revendication 1, dans lequel l'unité de régulation affiche la durée de vie restante dans le cas d'un mode de fonctionnement sans production de gaz de combustion et une durée de vie résiduelle dans le cas d'un mode de fonctionnement avec production de gaz de combustion.

5. Insufflateur selon au moins l'une des revendications 1 à 4, qui déclenche au moins une alarme lorsque la durée de fonctionnement restante déterminée du filtre (11) devient inférieure à une ou plusieurs durées prédéfinies.

6. Insufflateur selon la revendication 5, la durée d'un mode de fonctionnement sans production de gaz de combustion étant d'au moins 1,5 minute, de préférence d'environ 5 minutes, pour un débit volumique de 5 sLm et 50 sLm.

7. Insufflateur selon au moins l'une des revendications 1 à 6, dans lequel le filtre (11) comprend plusieurs couches, dont au moins une couche est une couche hydrofuge, dont au moins une couche est une couche filtrant les particules et dont au moins une couche est une couche à liaison chimique.

8. Insufflateur selon la revendication 7, dans lequel le filtre (11) comprend trois couches, la première couche dans le sens d'écoulement étant une couche hydrofuge, suivie d'une couche filtrant les particules et d'une couche à liaison chimique dans un ordre quelconque.

9. Insufflateur selon la revendication 8, dans lequel les fonctions de filtration des particules et de liaison chimique sont réalisées dans une seule couche.
